# EUROPÄISCHE PATENTSCHRIFT

(11) **EP 1 131 065 B1**
(45) Veröffentlichungstag und Bekanntmachung des Hinweises auf die Patenterteilung: **11.06.2003**
(21) Anmeldenummer: 99972532.8
(22) Anmeldetag: 29.10.1999
(51) Int. Cl.: A61K 31/194, A61P 37/06

(54) **DIALKYLFUMARATE ZUR BEHANDLUNG VON AUTOIMMUNERKRANKUNGEN**
DIALKYLFUMARATES FOR THE TREATMENT OF AUTOIMMUNE DISEASES
DIALKYLFUMARATES POUR LE TRAITEMENT DE MALADIES AUTOIMMUNES

(30) Priorität: 19.11.1998 DE 19853487
(43) Veröffentlichungstag der Anmeldung: 12.09.2001
(73) Patentinhaber: Fumapharm AG, 5630 Muri (CH)
(72) Erfinder: JOSHI, Rajendra, K., CH-8048 Zürich (CH); STREBEL, Hans-Peter, CH-5630 Muri (CH)
(74) Vertreter: Schwabe - Sandmair - Marx
(86) Internationale Anmeldenummer: EP9908215
(87) Internationale Veröffentlichungsnummer: WO00030622

(56) Entgegenhaltungen:
- EP-A- 0 188 749
- EP-A- 0 312 697
- CA-A- 2 248 955
- DE-A- 2 530 372
- HUNZIKER T. ET AL: "[Is psoriasis an autoimmune disease?]. PSORIASIS, EINE AUTOIMMUNKRANKHEIT ?." THERAPEUTISCHE UMSCHAU, (1993) 50/2 (110-113). , XP002088941
- M BACHARACH-BUHLES ET AL: "Fumaric Acid Esters (FAEs) Suppress CD 15- and ODP 4-positive Cells in Psoriasis" ACTA DERMATO-VENEREOLOGICA,XX,XX, Nr. SUPPL. 186, 16. September 1994 (1994-09-16), Seiten 79-82, XP002088940 ISSN: 0001-5555
- H M OCKENFELS ET AL: "The antipsoriatic agent dimethylfumarate immunomodulates T-cell cytokine secretion and inhibits cytokines of the psoriatic cytokine network" BRITISH JOURNAL OF DERMATOLOGY,XX,XX, Bd. 139, Nr. 3, 1. September 1998 (1998-09-01), Seiten 390-395, XP002089713 ISSN: 0007-0963
- "merck manual" 1987 , MERCK XP002141006 Seite 327, Absatz 2 - Absatz 6

## Beschreibung

Die Erfindung betrifft die Verwendung von Dialkylfumaraten zur Herstellung von pharmazeutischen Zubereitungen zur Anwendung in der Transplantationsmedizin oder zur Therapie von Autoimmunerkrankungen sowie Dialkylfumarate enthaltende pharmazeutische Zubereitungen in Form von Mikrotabletten oder Mikropellets.

Insbesondere betrifft sie also einerseits die Verwendung von Dialkylfumaraten zur Herstellung von pharmazeutischen Zubereitungen zur Behandlung, Verringerung oder Unterdrückung von Abstoßungsreaktionen des Empfängers gegen das Transplantat d.h. Host-versus-Graft-Reaktionen oder des Transplantats gegen den Empfänger d.h. Graft-versus-Host-Reaktionen. Andererseits betrifft sie die Verwendung von Dialkylfumaraten zur Herstellung pharmazeutischer Zubereitungen zur Behandlung von Autoimmunerkrankungen wie Polyarthritis, Multipler Sklerose, juvenilem Diabetes, der Hashimoto-Thyreoiditis, der Grave's disease (Graves Krankheit oder Basedow Krankheit), des systemischen Lupus erythematodes (SLE), des Sjögren Syndroms (Sjogren's Syndrome), der perniziösen Anämie, und der chronischen aktiven (=lupoiden) Hepatitis.

Transplantatabstoßung und Autoimmunerkrankungen beruhen beide auf medizinisch unerwünschten Reaktionen bzw. Fehlsteuerungen des Immunsystems. Wesentliche Mediatoren zur Beeinflussung desselben sind die Zytokine, wie die Interleukine oder der Tumornekrosefaktor α (TNF-α). Beide werden übicherweise durch Verabreichung von Immunsuppressiva wie Cyclosporin therapiert.

Autoimmunerkrankungen lassen sich als Ergebnis des Zusammenbruchs der Toleranz gegenüber körpereigenen Stoffen bzw. Antigenen auffassen. Diese bleibt im allgemeinen nur erhalten, wenn die Antigene immer wieder mit Immunzellen in Kontakt kommen. Bei Verlust der Toleranz kommt es zur Bildung von Autoantikörpern also einer fumaralen Immunantwort gegen körpereigenes Gewebe. Wie TNF-α hieran beteiligt ist, ist nicht bekannt.

Transplantationen sind Gewebe- bzw. Organverpflanzungen, d.h. Übertragungen von Geweben wie Hornhaut, Haut, Knochen (Knochenspänen), Gefäßen oder Faszien, von Organen, wie Niere, Herz, Leber, Lunge, Bauchspeicheldrüse oder Darm oder auch einzelner Zellen wie Inselzellen, Alphazellen und Leberzellen, worunter der Niere als transplantiertem Organ die wichtigste Bedeutung zukommt.

Nach dem Verwandschaftsgrad von Spender und Empfänger unterscheidet man die Autotransplantation (Übertragung auf einen anderen Körperteil des gleichen Individuums), die Isotransplantation (Übertragung auf ein anderes, genetisch gleiches Individuum) und die allogene Transplantation (Übertragung auf ein anderes Individuum der gleichen Art). Nach Herkunfts- und Transplantationsort unterscheidet man weiterhin die homotope Transplantation (Übertragung auf den gleichen Ort) und die heterotope Transplantation (Übertragung auf einen anderen Ort). Den genannten Transplantationen kommt in der modernen Medizin eine bedeutende Rolle zu.

Probleme bereitet in der Transplantationsmedizin vor allem die Transplantatabstoßung (Englisch: graft-rejection) nach der Transplantation des Gewebes, Organs oder der Zellen durch Immunabwehrreaktionen des Empfängers. Die Transplantatabstoßung wird auch Host-versus-Graft-Reaktion genannt. Die immunologischen Abwehrreaktion des Organismus gegen das Fremdprotein führt oft zur Abstoßung oder Auflösung der Transplantate. Bei den Host-versus-Graft-Reaktionen lassen sich verschiedene Stufen unterscheiden. Und zwar erfolgt die Reaktion je nach Grad der Verschiedenheit zwischen Empfänger und Spender unterschiedlich schnell, so daß man von akuter, subakuter oder chronischer Reaktion spricht. Die akut verlaufende Abstoßungsreaktion geht mit dem irreversiblen, medikamentös nicht beeinflußbaren Verlust (Nekrotisierung) des Transplantats in Folge Arteriitis bzw. Arteriolitis innerhalb von 48 Stunden einher. Die subakute Abstoßungsreaktion zeigt sich als Abstoßungskrise ab dem 12. Tag bis zum 4. Monat mit reversiblen Funktionsstörungen in Folge Transplantatvaskulopathie. Schließlich wird als chronische Abstoßungsreaktion der über Wochen bis Jahre fortschreitende, medikamentös kaum be-einflußbare Funktionverlust des Transplantats in Folge vaskulärer Veränderungen bspw. obliterierender Arteriopathie bezeichnet.

Umgekehrt kann es bei der Transplantation von immunkompetenten Geweben, d. h. vor allem bei Knochenmarkstransplantationen, zu Abstoßungsreaktionen des Transplantats gegen den Empfänger kommen, den sogenannten Graft-versus-Host-Reaktionen. Auch hier ist nach der Schwere der Reaktion einzuteilen und treten im wesentlichen ähnliche Komplikationen wie bei der Host-versus-Graft-Reaktion auf, nämlich Artheriopathien und Nekrosen.

Zur Vermeidung dieser Abstoßungsreaktionen d.h. der Host-versus-Graft-Reaktion sowie der Graft-versus-Host-Reaktion bedient sich die Transplantationsmedizin im wesentlichen der Immunsuppression, also der Abschwächung der normalen Immunantwort. Hierzu setzt man häufig Anti-Lymphozytenseren in Kombination mit Corticosterioden und sogenannten Antimetaboliten bspw. Purinanaloga wie 6-Mercaptopurin und Thioguanin ein, die die Nukleinsäure- und Proteinsynthese stören und damit die Zellteilung und Proliferation verhindern. Dies führt zur Unterdrückung der Antikörperproduktion und der zellulären Immunantwort. Bei den zur Therapie verwendeten Immunsupressiva handelt es sich um Substanzen, die die Immunreaktion im Körper entweder spezifisch oder unspezifisch unterdrücken bzw. abschwächen. Unspezifische Immunsuppresiva sind Zytostatika wie bspw. Alkylantien oder Antimetabolite.

Daneben kennt man Wirkstoffe, die zumindest teilweise eine spezifische Immunsuppression bewirken wie Corticosteriode, Antiseren, Antikörper FK-506, Tacrolimus, Mycophenolatmofetil und hauptsächlich die Cyclosporine, wie Cyclosporin A. Durch Einsatz moderner Immunsupressiva, für die als wichtigste Vertreter die Cyclosporine, insbesondere Cyclosporin A zu nennen sind, konnten die Transplantationsresultate in den letzten Jahren erheblich verbessert werden. Die 1-Jahres-Überlebensrate beträgt zur Zeit für Lebertransplantationen etwa 60 %, für Herztransplantationen ca. 80 % und für Nierentransplantationen über 90 %.

Der Graft-versus-Host-Reaktion vergleichbar sind Autoimmunerkrankungen, bei denen nunmehr das körpereigene Immunsystem ebenfalls körpereigene Organe, Gewebe und Zellen angreift. Auch hier handelt es sich um eine medizinisch unerwünschte Reaktion des Immunsystems, die ebenfalls mit Immunsuppressiva therapiert werden kann.

Die Gefahr bei der Anwendung von Immunsuppressiva liegt in der Schwächung der körpereigenen Abwehr gegen Infektionskrankheiten und dem erhöhten Risiko maligner Erkrankungen. Die Aufgabe der Erfindung besteht daher darin, eine pharmazeutische Zubereitung zur Verwendung in der Transplantationsmedizin bereitzustellen, welche zur Behandlung, insbesondere Unterdrückung, Abschwächung und/oder Linderung der Host-versus-Graft-Reaktionen und Graft-versus-Host-Reaktionen verwendet werden kann, diesen Nachteil jedoch nicht aufweist.

Eine weitere Aufgabe der Erfindung besteht darin, eine pharmazeutische Zubereitung bereitzustellen, die zur Behandlung von Autoimmunerkrankungen wie insbesondere der Polyarthritis, der Multiplen Sklerose, der juvenilen Diabetes, der Hashimoto-Thyreoiditis, der Grave's disease (Graves Krankheit oder Basedow Krankheit), der systemischen Lupus erythematodes (SLE), des Sjögren Syndroms (Sjogren's Syndrome), der perniziösen Anämie; und der chronischen aktiven (=lupoiden) Hepatitis verwendet werden kann, ohne die Nachteile der Immunsuppression zu zeigen.

Die Lösung der erfindungsgemäßen Aufgabe liegt in der Verwendung bestimmter Dialkylfumarate zur Herstellung von pharmazeutischen Zubereitungen zur Verwendung in der Transplantationsmedizin und zur Therapie von Autoimmunerkrankungen sowie durch diese Dialkylfumarate enthaltende pharmazeutischen Zubereitungen in Form von Mikrotabletten und Mikropellets. Die erfindungsgemäßen Gegenstände sind in den Ansprüchen im einzelnen gekennzeichnet. Die erfindungsgemäßen Zubereitungen enthalten keine freie Fumarsäure per se.

Bekannt ist, daß pharmazeutische Zubereitungen, die nach Verabreichung bei ihrem biologischen Abbau in den Zitronensäurezyklus einmünden oder diesem angehören, zumeist in hoher Dosierung immer mehr an therapeutischem Wert gewinnen, da man mit ihrer Hilfe kryptogenetisch bedingte Krankheiten zu lindern oder zu heilen vermag.

So hemmt Fumarsäure das Wachstum des Ehrlich-Ascites-Tumors bei Mäusen, vermindert die toxischen Effekte von Mitoycin C und Aflatoxin und besitzt eine antipsoriatische sowie antimikrobielle Wirkung. Hohe Verabreichungsdosen von Fumarsäure oder ihren bisher bekannten Derivaten wie Dihydroxylfumarsäure, Fumaramid und Fumaronitril besitzen bei parenteraler, dermaler, insbesondere aber peroraler Verabreichung eine derart unzumutbare Nebenwirkungsrate und hohe Toxität, daß bisher meist von einer solchen Therapie abgesehen werden mußte.

Untersuchungen der Anmelderin haben überraschend gezeigt, daß Methylhydrogenfumarat ein Metabolit des Dimethylfumarats die Endotoxin-stimulierte TNF-α Sekretion in menschlichen mononuclearen Zellen des Peripheriebluts (periphere blood mononuclear cells = PBMC-Zellen) und in isolierten Monozyten initial steigert. Weiterhin konnte gezeigt werden, daß Fumarsäure dem Cyclosporin vergleichbare Wirkung auf die in-vitro- und invivo-Hämagglutination hat.

Überraschend wurde nun gefunden, daß Dialkylfumarate vorteilhaft zur Herstellung pharmazeutischer Zubereitungen zur Verwendung in der Transplantationsmedizin und zur Therapie von Autoimmunerkrankungen verwendet werden können. Die diese Dialkyl-fumarate enthaltenden Zubereitungen gestatten nämlich in überraschender Weise eine positive Modulation des Immunsystems bei Host-versus-Graft-Reaktionen sowie Graft-versus-Host-Reaktionen und anderen Autoimmunerkrankungen.

In der europäischen Patentanmeldung 18 87 49 sind bereits Fumarsäurederivate und sie enthaltende pharmazeutische Zubereitungen zur Behandlung der Psoriasis beschrieben. Aus der DE-A-25 30 372 sind pharmazeutische Zubereitungen zur Behandlung der Psoriasis bekannt, die eine Mischung aus Fumarsäure und weiteren Fumarsäurederivaten enthalten. Der Anteil an freier Fumarsäure ist bei diesen Arzneimitteln obligatorisch.

Die DE-A-26 21 214 beschreibt Arzneimittel zur Behandlung der Psoriasis, die den Fumarsäuremonoethylester und dessen Mineralsalze als Wirkstoff enthalten. Aus der Publikation "Hautarzt (1987) 279-285" ist die Verwendung von Fumarsäuremonoethylestersalzen bekannt. Aus dem Patent EP 0 312 697 B1 sind pharmazeutische Zubereitungen zur Behandlung der Psoriasis, psoriatischer Arthritis, Neurodermitis und Enteritis regionalis Crohn bekannt, die eine Mischung aus Fumarsäuremonoalkylestersalzen und einem Fumarsäurediester enthalten.

Genauer wird die erfindungsgemäße Aufgabe gelöst durch Verwendung von einem oder mehreren Dialkylfumaraten der Formel: 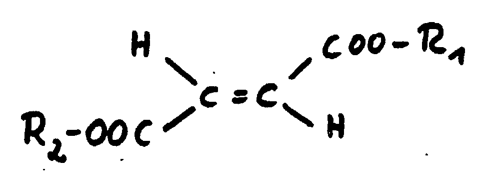 in der R₁ und R₂, die gleich oder verschieden sein können, unabhängig voneinander einen linearen, verzweigten oder cyclischen, gesättigten oder ungesättigten C₁₋₂₀-Alkylrest, der gegebenenfalls mit Halogen (Cl, F, J, Br), Hydroxy, C₁₋₄-Alkoxy, Nitro oder Cyano substituiert sein kann, darstellen zur Herstellung einer pharmazeutischen Zubereitung zur Verwendung in der Transplantationsmedizin oder zur Therapie von Autoimmunerkrankungen.

Bei den C₁₋₂₀-Alkylresten, bevorzugt C₁₋₈-Alkylresten, am meisten bevorzugt C₁₋₅-Alkylresten, handelt es sich beispielsweise um Methyl, Ethyl, n-Propyl, Isopropyl, n-Butyl, sec-Butyl, t-Butyl, Pentyl, Cyclopentyl, 2-Ethylhexyl, Hexyl, Cyclohexyl, Heptyl, Cycloheptyl, Octyl, Vinyl, Allyl, 2-Hydroxyethyl, 2- oder 3-Hydroxypropyl, 2-Methoxyethyl, Methoxymethyl, 2- oder 3-Methoxypropyl. Vorzugsweise ist mindestens einer der Reste R₁ oder R₂ ein C₁₋₅-Alkyl, insbesondere Methyl oder Ethyl. Stärker bevorzugt sind R₁ und R₂ gleiche oder verschiedene C₁₋₅-Alkylreste wie Methyl, Ethyl, n-Propyl oder t-Butyl, wobei Methyl und Ethyl besonders bevorzugt sind. Am meisten bevorzugt sind R₁ und R₂ identisch Methyl oder Ethyl. Ganz besonders bevorzugt sind das Dimethylfumarat, das Methylethylfumarat und das Diethylfumarat.

Die erfindungsgemäß zu verwendenden Dialkylfumarate werden gemäß im Stand der Technik bekannter Verfahren hergestellt (siehe beispielsweise EP 0 312 697).

Bevorzugt werden die Wirkstoffe zur Herstellung oraler Zubereitungen in Form von Tabletten, von Mikrotabletten, Pellets oder Granulaten gegebenenfalls in Kapseln oder Sachets verwendet. Hiervon sind Zubereitungen in Form von Mikrotabletten oder Pellets, gegebenenfalls abgefüllt in Kapseln oder Sachets bevorzugt und stellen ebenfalls Gegenstände der Erfindung dar. Die oralen Zubereitungen können mit einem magensaftresistenten Überzug versehen sein. Kapseln können Weich- oder Hartgelatine-Kapseln sein.

Die erfindungsgemäßen verwendeten Dialkylfumarate können allein oder als Gemisch mehrerer Verbindungen, gegebenenfalls in Kombination mit üblichen Träger- und Hilfsstoffen verwendet werden. Die zu verwendenden Mengen sind so bemessen, daß die erhaltenen Zubereitungen eine 10 bis 300 mg Fumarsäure entsprechende Menge an Wirkstoff enthalten.

Bevorzugte Zubereitungen gemäß der Erfindung enthalten Dimethylfumarat und/oder Diethylfumarat in einer Gesamtmenge von 10 bis 300 mg.

Nach einer vorzugsweise Ausgestaltung liegt die Größe bzw. der mittlere Durchmesser der Pellets oder Mikrotabletten im Bereich von 300 bis 2000 µm, insbesondere im Be-reich von 500 µm oder 1000 µm.

An zu therapierenden Autoimmunerkrankungen sind parallel zur Graft-versus-Host-Reaktion (siehe oben) folgende Erkrankungen zu nennen: Polyarthritis, Multiple Sklerose, Graft-versus-Host-Reaktionen, juvenile Diabetes, Hashimoto-Thyreoiditis, Grave's disease (Graves Krankheit oder Basedow Krankheit), systemischen Lupus erythematodes (SLE), Sjögren Syndroms (Sjogren's Syndrome), perniziösen Anämie und die chronische aktive (=lupoide) Hepatitis.

Pharmazeutische Zubereitungen zur Behandlung dieser Erkrankungen sind neben den oben genannten Zubereitungen für die perorale Verabreichung in Form von Mikropellets, Mikrotabletten, Kapseln (beispielsweise Weich- oder Hartgelatinekapseln), Granulaten und Tabletten Zubereitungen zur kutanen und transdermalen Verabreichung in Form von Salben, Pflastern, Lotionen und Duschmitteln sowie für die parenterale Verabreichung in Form wäßriger Mikro-Dispersionen, Ö/W-Emulsionen oder öligen Lösungen zur rektalen Verabreichung von Suppositorien oder Mikroklistieren. Pharmazeutische Zubereitungen in Form von Mikrotabletten oder Mikropellets sind zur Therapie aller vorstehend genannten Autoimmunerkrankungen, sowie zusätzlich der Psoriasis, der psoriatrischen Arthritis, der Neurodermitis sowie der Enteritis regionalis Crohn bevorzugt und stellen ebenfalls einen Gegenstand der Erfindung dar.

Erfindungsgemäß kann die Therapie mit Dialkylfumaraten auch in Kombination mit einem oder mehreren Präparaten der bei Organtransplantationen üblicherweise eingesetzten Tripledrugtherapie oder auch alleine mit Cyclosporin A erfolgen. Hierzu können die verabreichten Präparate eine Kombination der Wirkstoffe in den bekannten Dosierungen bzw. Mengen enthalten. Ebenso kann die Kombinationstherapie in der parallen Verabreichung separater Präparate auf gleichem oder unterschiedlichen Applikationsweg bestehen. Gegebenenfalls kann die Dosis des neben der erfindungsgemäß verabreichten Fumarsäurederivatdosis enthaltenen Wirkstoffs in vorteilhafter Weise gesenkt werden.

Eine weitere Ausgestaltung der erfindungsgemäßen Verwendung besteht darin, die Arzneitherapie mit Immunsuppressiva wie Cyclosporin sequentiell mit einer Applikation der oben bezeichneten Dialkylfumarate zu alternieren. Das heißt, daß nach ein- bis mehrwöchiger Cyclosporin-Therapie eine ein- bis mehrwöchige Applikation von Fumarsäurederivaten gemäß vorstehender Bedeutung erfolgen kann. Dadurch läßt sich die Dosierung von Cyclosporin A senken, wodurch eine bedeutend verringerte Nebenwirkungsrate bei der Langzeittherapie erreicht werden kann.

Durch Verabreichung der Dialkylfumarate in Form von Mikrotabletten, die bevorzugt ist, lassen sich die bei der Verabreichung von herkömmlichen Tabletten zwar bereits gegenüber Fumarsäurederivaten bzw. -salzen verminderten, aber immer noch beobachteten, gastro-intestinalen Reizungen bzw. Nebenwirkungen weiter verringern.

Vermutlich werden bei Verabreichung herkömmlicher Tabletten die Inhaltsstoffe der Tablette lokal in zu hoher Konzentration im Darm freigesetzt, wodurch eine örtliche Reizung der Darmschleimhaut verursacht wird. Durch diesen lokalen Reiz werden kurzfristig sehr hohe Konzentrationen an TNF-α freigesetzt, welche für die gastro-intestinalen Nebenwirkungen verantwortlich sein könnten. Bei der Applikation von magensaftresistenten Mikrotabletten in Kapseln werden dagegen lokal niedrige Konzentrationen der Wirkstoffe an den Darmepithelzellen erzielt. Die Mikrotabletten werden vom Magen nach und nach durch die Magenperistaltik in den Dünndarm abgegeben und es kommt zu einer besseren Verteilung der Wirkstoffe.

Magensaftresistente Mikrotabletten der gleichen Dosierung verteilen sich also bereits im Magen und gelangen daher bereits portionsweise in den Darm, wo die Wirkstoffe in kleineren Dosen freigesetzt werden. So wird die lokale Reizung der Darmepithelzellen vermieden, ebenso wie die Freisetzung von TNF-α. Hieraus resultiert vermutlich die bessere Magen-Darmverträglichkeit von Mikrotabletten gegenüber herkömmlichen Tabletten.

Weiterhin wird die Resorption verbessert, da es sich bei den erfindungsgemäß zu verwendenden Dialkylfumaraten nicht um den eigentlichen Wirkstoff, sondern um ein sogenanntes Pro-drug handelt, welches erst im Körper zum eigentlichen Wirkstoff umgewandelt werden muß.

Im folgenden werden zur Erläuterungen der erfindungsgemäßen Verwendung verschiedene Beispiele für die Herstellung bevorzugter Arzneimittel gegeben:

### Herstellungsbeispiele

Grundsätzlich können die erfindungsgemäßen oralen Zubereitungen in Form von Tabletten oder Mikrotabletten nach klassischen Tablettierverfahren hergestellt werden. Anstelle dieser klassischen Tablettiermethoden können auch andere Methoden zur Herstellung von Tabletten angewendet werden, wie Direkttablettierung sowie Verfahren zur Herstellung fester Dispersionen nach der Schmelzmethode und der Sprühtrocknungsmethode.

Die Tabletten können mit magensaftresistenten Überzügen versehen sein. Der magensaftresistente Überzug kann in einem klassischen Dragierkessel aufgeleert oder aufgesprüht sowie in einer. Wirbelschichtapparatur erfolgen. Weiterhin kann die Tablette mit einem Filmcoat versehen werden.

### Beispiel 1

### Herstellung von magensaftresistenten Mikrotabletten in Kapseln, enthaltend 120,0 mg Dimethylfumarat, entsprechend 96 mg Fumarsäure

12,000 kg Dimethylfumarat werden zerkleinert, und mittels eines Siebes 800 unter entsprechenden Vorsichtsmaßnahmen (Atemmaske, Handschuhe, Schutzanzug etc.) homogenisiert. Es wird ein Hilfsstoffgemisch folgender Zusammensetzung hergestellt: 17,50 kg Stärkederivat (STA-RX® 1500), 0,30 kg Cellulose mikrokristallin (Avicel® PH 101), 0,75 kg PVP (Kollidon® 120), 4,00 kg Primogel®, 0,25 kg Kieselsäure kollodial (Aerosil®). Das gesamte Pulvergemisch wird mit dem Wirkstoff versetzt, gemischt, mittels eines Siebes 200 homogenisiert und mit einer 2 %-igen wäßrigen Lösung von Polyvinylpyrrolidon (Kollidon® K25) auf übliche Weise zu einem Bindemittelgranulat verarbeitet und in trockenem Zustand mit der äußeren Phase gemischt. Diese besteht aus 0,50 kg Mg-Stearat und 1.50 kg Talkum.

Das Pulvergemisch wird anschließend auf übliche Weise zu gewölbten Tabletten von 10,0 mg Bruttomasse und 2,0 mm Durchmesser gepreßt.

Zum Erreichen der Magensaftresistenz wird hier bspw. portionenweise eine Lösung von 2,250 kg Hydroxypropylmethylcellulosephthalat (HPMCP, Pharmacoat® HP 50) in einem Gemisch folgender Lösungsmittel aufgelöst: Aceton 13,00 l, Ethanol (94 Gew.-% denaturiert mit 2 % Keton) 13,50 l und Aqua demineralisata 1,50 l. Zu der fertigen Lösung wird als Weichmacher Rizinusöl (0.240 kg) zugegeben und auf übliche Weise in Portionen auf die Tablettenkerne aufgetragen.

Nach beendeter Trocknung wird anschließend in der gleichen Apparatur eine Suspension folgender Zusammensetzung als Filmcoat aufgetragen: Talk 0,340 kg, Titan-(VI)-oxid Cronus RN 56: 0.400 kg, Farblack L-Rotlack 86837: 0.324 kg, Eudragit E 12,5 %: 4,800 kg und Polyethylenglycol 6000 pH 11 XI: 0,120 kg in einem Lösungsmittelgemisch folgender Zusammensetzung: 2-Propanol 8,170 kg, Aqua demineralisata 0,200 kg und Glycerintriacetat (Triacetin) 0,600 kg. Die magensaftresistenten Mikrotabletten werden anschließend in Hartgelatine-Steckkapseln zu 400 mg Nettorgewicht eingefüllt und verschlossen.

### Beispiel 2

### Herstellung von magensaftresistenten Mikrotabletten in Kapseln, enthaltend 120,0 mg Dimethylfumarat, entsprechend 96 mg Fumarsäure

12,000 kg Dimethylfumarat werden zerkleinert und wie oben homogenisiert. Es wird ein Hilfsstoffgemisch folgender Zusammensetzung hergestellt: 23,20 kg mikrokristalline Cellulose (Avicel® PH 200), 3,00 kg Croscarmelose Natrium (AC-Di-SOL-SD-711), 2,50 kg Talkum, 0,10 kg Siliciumdioxid wasserfrei (Aerosil® 200) und 1,00 kg Mg-Stearat. Das gesamte Pulvergemisch wird mit dem Wirkstoff versetzt und homogen gemischt. Das Pulvergemisch wird anschließend mittels Direkttablettierung zu gewölbten Tabletten von 10,0 mg Bruttomasse und 2,0 mm Durchmesser gepreßt.

Anschließend wird eine Lösung von 0,94 Eudragit® L in Isopropanol hergestellt, die zusätzlich 0,07 kg Dibutylphthalat enthält. Diese Lösung wird auf die Tablettenkerne aufgesprüht. Danach wird eine Dispersion von 17,32 kg Eudragit® L D-55 und einer Mischung aus 2,80 kg Mikrotalkum, 2,00 kg Macrogol 6000 und 0,07 kg Dimetican in Wasser hergestellt und auf die Kerne aufgesprüht.

Die magensaftresistenten Mikrotabletten werden anschließend in Hartgelatine-Steckkapseln zu 650 mg Nettogewicht eingefüllt und verschlossen.

### Beispiel 3

### Herstellung von Mikropellets in Kapseln enthaltend 50,0 mg Dimethylfumarat, entsprechend 40 mg Fumarsäure

5,000 kg Dimethylfumarat werden zerkleinert und wie oben homogenisiert. Daneben werden 2 Liter einer 20 %-igen (m/V) Polyvinylpyrrolidon-Lösung (Kollidon K-30) in Ethanol vorbereitet. 7,250 kg Nonpareilles Pellets werden in einem Dragierkessel vorgelegt und mit einem Teil der Kollidon K-30 Lösung besprüht bis diese leicht feucht werden. Der Wirkstoff wird danach portionsweise zugegeben bis zum Auftrocknen der Pellets. Diese Vorgehensweise der Befeuchtung/Auftrocknung wird bis zur endgültigen Zugabe des Wirkstoffgemisches weitergeführt. Die Pellets werden letztlich bis zum vollständigen Austrocknen bewegt.

Die Pellets werden danach in Hartgelatinekapseln abgefüllt (126,5 mg Pellets/Kapsel).

### Beispiel 4

### Herstellung von magenasaftresistenten Kapseln, enthaltend 110.0 mg Dimethylfumarat, entsprechend 88 mg Fumarsäure.

11,000 kg Dimethylfumarat werden mit einem Gemisch, bestehend aus 14,00 kg Stärke, 5,65 kg Lactose, 2,00 kg mikrokristalline Cellulose (Avicel®, 1,00 kg Polyvinylpyrrolidon (Kollidon® 25) und 2,443 kg Primogel®. intensiv gemischt und unter entsprechenden Vorsichtsmaßnahmen (Atemmaske, Handschuhe, Schutzanzug etc.) mittels eines Siebes 800 homogenisiert.

Das gesamte Pulvergemisch wird mit einer 2 %-igen wässrigen Lösung von Polyvinylpyrrolidon (Kollidon® K25) auf übliche Weise zu einem Bindemittelgranulat verarbeitet und in getrocknetem Zustand mit der äußeren Phase gemischt. Diese besteht aus 0,350 kg kolloidaler Kieselsäure (Aerosil®), 0,500 kg Mg-Stearat und 1,500 kg Talkum. Das homogene Gemisch wird anschließend in entsprechende Kapseln in Portionen von 400 mg abgefüllt. welche darauf dann auf übliche Weise mit einem magensaftresistenten Überzug, bestehend aus Hydroxypropylmethylcellulosestearat und Rizinusöl als Weichmacher, versehen werden. Die Abfüllung kann anstelle von Hartgelatinekapseln auch in entsprechende magensaftresistente Kapseln, bestehend aus einem Gemisch von Celluloseacetatphthalat (CAP) und Hydroxypropylmethylcellulosephthalat (HPMCP) erfolgen.

Die Arzneitherapie im Zusammenhang mit den erfindungsgemäßen Indikationen ist im Vergleich zur Behandlung mit Stoffen des Standes der Technik, beispielsweise Cyclosporin, das zu massiven Nierenstörungen oder Erkrankungen des lymphoproliferativen Systems führen kann, wogegen Fumarsäurederivate nur selten zu ernstlicheren Nebenwirkungen führen.

Der immunsuppressive Effekt von Cyclosporin ist unter anderem bedingt durch eine Hemmung der Bildung von Th-1-Zellen. Wie in-vitro-Versuche der Anmelderin gezeigt haben, bewirken Fumarate eine Verschiebung des Zytokinmusters vom Typ Th1 zum Typ Th2 Zytokinmuster.

Dieser unerwartete Effekt der erfindungsgemäßen Verwendung ist insbesondere in Anbetracht der stets notwendingen Langzeittherapie und -prophylaxe der Graft-versus-Host-Reaktionen oder der Host-versus-Graft-Reaktionen oder anderer Autoimmunerkrankungen wie der Multiplen Sklerose von höchstem Interesse. Die erfindungsgemäße Verwendung ist darüber hinaus auch bei der Substitution der bekanntermaßen mit starken Nebenwirkungen verbundenen Kortikoidtherapie von Autoimmunerkrankungen von größter Bedeutung.

## Patentansprüche

1. Verwendung eines oder mehrerer Dialkylfumarate zur Herstellung einer pharmazeutischen Zubereitung zur Behandlung von Host-versus-Graft-Reaktionen oder Graft-versus-Host-Reaktionen bei Organ- und Zelltransplantationen.

2. Verwendung eines oder mehrerer Dialkylfumarate zur Herstellung einer pharmazeutischen Zubereitung zur Behandlung von Autoimmunerkrankungen ausgewählt aus der Gruppe, bestehend aus juvenilem Diabetes, der Hashimoto-Thyreoiditis, der Grave's disease (Graves Krankheit oder Basedow Krankheit), des systemischen Lupus erythematodes (SLE), des Sjögren Syndroms (Sjogren's Syndrome), der perniziösen Anämie und der chronischen aktiven (=lupoiden) Hepatitis.

3. Verwendung gemäß Anspruch 1 oder 2 von einem oder mehreren Dialkylfumaraten der Formel: 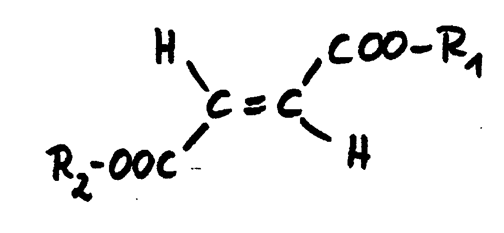 in der R₁ und R₂, die gleich oder verschieden sein können, unabhängig voneinander einen linearen, verzweigten oder cyclischen, gesättigten oder ungesättigten C₁₋₂₀-Alkylrest, der gegebenenfalls mit Halogen (Cl, F, J, Br), Hydroxy, C₁₋₄-Alkoxy, Nitro oder Cyano substituiert sein kann, darstellen, zur Herstellung einer pharmazeutischen Zubereitung zur Verwendung in der Transplantationsmedizin oder zur Therapie von Autoimmunerkrankungen ausgewählt aus der Gruppe, bestehend aus juvenilem Diabetes, der Hashimoto-Thyreoiditis, der Grave's disease (Graves Krankheit oder Basedow Krankheit), des systemischen Lupus erythematodes (SLE), des Sjögren Syndroms (Sjogren's Syndrome), der perniziösen Anämie und der chronischen aktiven (=lupoiden) Hepatitis.

4. Verwendung gemäß Anspruch 3, **dadurch gekennzeichnet, dass** es sich bei den Resten R₁ und R₂ um Methyl, Ethyl, n-Propyl, Isopropyl, n-Butyl, sec-Butyl, t-Butyl, Pentyl, Cyclopentyl, 2-Ethylhexyl, Hexyl, Cyclohexyl, Heptyl, Cycloheptyl, Octyl, Vinyl, Allyl, 2-Hydroxyethyl, 2- und/oder 3-Hydroxypropyl, 2-Methoxyethyl, Methoxyethyl oder 2- oder 3-Methoxypropyl handelt.

5. Verwendung nach Anspruch 3 oder 4, **dadurch gekennzeichnet, dass** R₁ und R₂ identisch sind und Methyl oder Ethyl darstellen.

6. Verwendung nach einem der Ansprüche 1 bis 5, bei der die Wirkstoffe zu einer oralen Zubereitung in Form von Tabletten, von Mikrotabletten, Pellets oder Granulaten gegebenenfalls in Kapseln oder Sachets formuliert werden.

7. Verwendung nach Anspruch 6, **dadurch gekennzeichnet, dass** die Größe bzw. der mittlere Durchmesser von Pellets oder Mikrotabletten im Bereich von 300 bis 2000 µm liegt.

8. Verwendung nach den Ansprüchen 1 bis 7, **dadurch gekennzeichnet, dass** die Zubereitung in Form von Weich- oder Hartgelatinekapseln vorliegt.

9. Verwendung nach einem der Ansprüche 1 bis 8, bei der die Zubereitung eine 10 bis 300 mg Fumarsäure entsprechende Menge an Wirkstoff enthält.

10. Verwendung nach den Ansprüchen 6 bis 9, **dadurch gekennzeichnet, dass** die Dosiseinheiten der Arzneimittel mit einem magensaftresistenten Überzug versehen sind.

11. Verwendung nach einem der Ansprüche 1 bis 5, **dadurch gekennzeichnet, dass** die Arzneimittel in Form von Präparaten für die kutane und transdermale Verabreichung, Präparaten für die parenterale Verabreichung und Präparaten für die rektale Verabreichung eingesetzt werden.

12. Pharmazeutische Zubereitung in Form von Mikrotabletten oder Pellets, bestetend aüs einem oder mehreren Dialkylfumaraten der Formel: 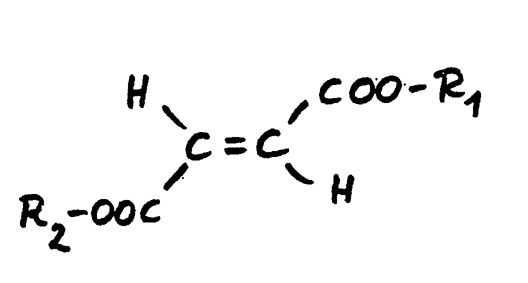 in der R₁ und R₂, die gleich oder verschieden sein können, unabhängig voneinander einen linearen, verzweigten oder cyclischen, gesättigten oder ungesättigten C₁₋₂₀-Alkylrest, der gegebenenfalls mit Halogen (Cl, F, J, Br), Hydroxy, C₁₋₄-Alkoxy, Nitro oder Cyano substituiert sein kann, darstellen, und
gegebenenfalls geeigneten Träger- und. Hilfsstoffen.

13. Zubereitung gemäß Anspruch 12, enthaltend Dimethylfumarat, Diethylfumarat oder Methylethylfumarat.

14. Zubereitung gemäß Anspruch 12 oder 13, enthaltend eine 10 bis 300 mg Fumarsäure entsprechende Menge an Wirkstoff.

15. Verwendung eines oder mehrerer Dialkylfumarate zur Herstellung einer pharmazeutischen Zubereitung in Form von Mikrotabletten oder Pellets zur Therapie von Psoriasis, des psoriatrischen Arthritis, der Naürodermitis, der Enteritis regionalis Crohn sowie von Autoimmunerkrankungen ausgewählt aus der Gruppe, bestehend aus Polyarthritis, Multipler Sklerose, juvenilem Diabetes, der Hashimoto-Thyreoiditis, der Grave's disease (Graves Krankheit oder Basedow Krankheit), des systemischen Lupus erythematodes (SLE), des Sjögren Syndroms (Sjogren's Syndrome), der perniziösen Anämie und der chronischen aktiven (=lupoiden) Hepatitis.

16. Verwendung gemäß Anspruch 15 von einem oder mehreren Dialkylfumaraten der Formel: 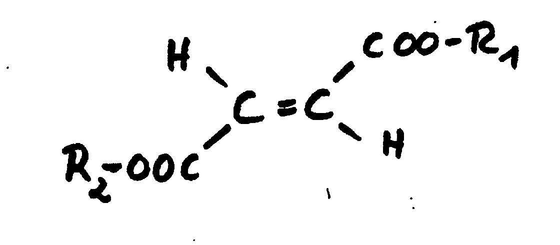 in der R₁ und R₂, die gleich oder verschieden sein können, unabhängig voneinander einen linearen, verzweigten oder cyclischen, gesättigten oder ungesättigten C₁₋₂₀-Alkylrest, der gegebenenfalls mit Halogen (Cl, F, J, Br), Hydroxy, C₁₋₄-Alkoxy, Nitro oder Cyano substituiert sein kann, darstellen, zur Herstellung einer pharmazeutischen Zubereitung zur Therapie von Psoriasis, des psoriatrischen Arthritis, der Naürodermitis, der Enteritis regionalis Crohn sowie von Autoimmunerkrankungen ausgewählt aus der Gruppe, bestehend aus Polyarthritis, Multipler Sklerose, juvenilem-Diabetes, der Hashimoto-Thyreoiditis, der Grave's disease (Graves Krankheit oder Basedow Krankheit), des systemischen Lupus erythematodes (SLE), des Sjögren Syndroms (Sjogren's Syndrome), der perniziösen Anämie und der chronischen aktiven (=lupoiden) Hepatitis.

17. Verwendung gemäß Anspruch 16, **dadurch gekennzeichnet, dass** es sich bei den Resten R₁ und R₂ um Methyl, Ethyl, n-Propyl, Isopropyl, n-Butyl, sec-Butyl, t-Butyl, Pentyl, Cyclopentyl, 2-Ethylhexyl, Hexyl, Cyclohexyl, Heptyl, Cycloheptyl, Octyl, Vinyl, Allyl, 2-Hydroxyethyl, 2- und/oder 3-Hydroxypropyl, 2-Methoxyethyl, Methoxyethyl oder 2- oder 3-Methoxypropyl handelt.

18. Verwendung nach Anspruch 15 oder 16, **dadurch gekennzeichnet, dass** R₁ und R₂ identisch sind und Methyl oder Ethyl darstellen.

19. Verwendung nach einem der Ansprüche 15 bis 18, bei der die Wirkstoffe zu einer oralen Zubereitung in Form von Mikrotabletten oder Pellets in Kapseln oder Sachets formuliert werden.

20. Verwendung nach einem der Ansprüche 15 bis 19, **dadurch gekennzeichnet, dass** die Größe bzw. der mittlere Durchmesser von Pellets oder Mikrotabletten im Bereich von 300 bis 2000 µm liegt.

21. Verwendung nach Anspruch 19, **dadurch gekennzeichnet, dass** die Zubereitung in Form von Weich- oder Hartgelatinekapseln vorliegt.

22. Verwendung nach einem der Ansprüche 15 bis 21, bei der die Zubereitung eine 10 bis 300 mg Fumarsäure entsprechende Menge an Wirkstoff enthält.

23. Verwendung nach den Ansprüchen 19 bis 22, **dadurch gekennzeichnet, dass** die Pellets oder Mikrotabletten mit einem magensaftresistenten Überzug versehen sind.

## Claims

1. The use of one or more dialkyl fumarates for preparing a pharmaceutical preparation for treating host-versus-graft reactions or graft-versus-host reactions in organ and cell transplantation.

2. The use of one or more dialkyl fumarates for preparing a pharmaceutical preparation for the therapy of autoimmune diseases selected from the group consisting of juvenile-onset diabetes, Hashimoto's thyroiditis, Grave's disease, systemic Lupus erythematodes (SLE), Sjogren's syndrome, pernicious anaemia and chronic active (lupoid) hepatitis.

3. The use according to claim 1 or 2 of one or more dialkyl fumarates of the formula 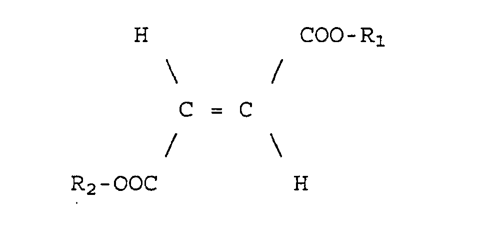 wherein R₁ and R₂, which may be the same or different, independently represent a linear, branched or cyclic, saturated or unsaturated C₁₋₂₀ alkyl radical which may be optionally substituted with halogen (Cl, F, I, Br), hydroxy, C₁₋₄ alkoxy, nitro or cyano for preparing a pharmaceutical preparation for use in transplantation medicine or for the therapy of autoimmune diseases selected from the group consisting of juvenile-onset diabetes, Hashimoto's thyroiditis, Grave's disease, systemic Lupus erythematodes (SLE), Sjogren's syndrome, pernicious anaemia and chronic active (lupoid) hepatitis.

4. The use according to claim 3, **characterised in that** the radicals R₁ and R₂ are methyl, ethyl, n-propyl, isopropyl, n-butyl, sec-butyl, t-butyl, pentyl, cyclopentyl, 2-ethyl hexyl, hexyl, cyclohexyl, heptyl, cycloheptyl, octyl, vinyl, allyl, 2-hydroxy ethyl, 2 and/or 3-hydroxy propyl, 2-methoxy ethyl, methoxy ethyl or 2- or 3-methoxy propyl.

5. The use according to claim 3 or 4, **characterised in that** R₁ and R₂ are identical and represent methyl or ethyl.

6. The use according to any of the claims 1 to 5 where the active ingredients are formulated into an oral preparation in the form of tablets, micro-tablets, pellets or granulates, optionally in capsules or sachets.

7. The use according to claim 6, **characterised in that** the size or the mean diameter, respectively, of the pellets or micro-tablets is in the range of 300 to 2,000 µm.

8. The use according to claims 1 to 7, **characterised in that** the preparation is present in the form of soft or hard gelatine capsules.

9. The use according to any of the claims 1 to 8 where the preparation contains an amount of the active ingredient corresponding to 10 to 300 mg of fumaric acid.

10. The use according to claims 6 to 9, **characterised in that** the dosage units of the drugs are provided with an enteric coating.

11. The use according to any of the claims 1 to 5, **characterised in that** the drugs are used in the form of preparations for cutaneous and transdermal administration, preparations for parenteral administration and preparations for rectal administration.

12. Pharmaceutical preparation in the form of micro-tablets or pellets consisting of one or more dialkyl fumarates of the formula 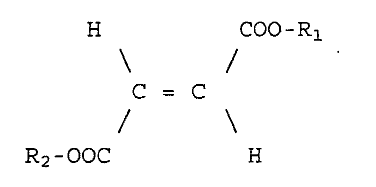 wherein R₁ and R_{2,} which may be the same or different, independently represent a linear, branched or cyclic, saturated or unsaturated C₁₋₂₀ alkyl radical which may be optionally substituted with halogen (Cl, F, I, Br), hydroxy, C₁₋₄ alkoxy, nitro or cyano,
and optionally suitable carriers and excipients.

13. A preparation according to claim 12 comprising dimethyl fumarate, diethyl fumarate or methylethyl fumarate.

14. A preparation according to claim 12 or 13 comprising an amount of the active ingredient corresponding to 10 to 300 mg of fumaric acid.

15. The use or one or more dialkyl fumarates for preparing a pharmaceutical preparation in the form of micro-tablets or pellets for the therapy of psoriasis, psoriatic arthritis, neurodermitis and enteritis regionalis Crohn, as well as of autoimmune diseases selected from the group consisting of polyarthritis, multiple sclerosis, juvenile-onset diabetes, Hashimoto's thyroiditis, Grave's disease, systemic Lupus erythematodes (SLE), Sjogren's syndrome, pernicious anaemia and chronic active (lupoid) hepatitis.

16. The use according to claim 15 of one or more dialkyl fumarates of the formula 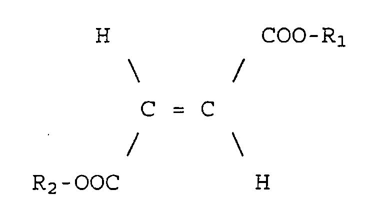 wherein R₁ and R₂, which may be the same or different, independently represent a linear, branched or cyclic, saturated or unsaturated C₁₋₂₀ alkyl radical which may be optionally substituted with halogen (Cl, F, I, Br), hydroxy, C₁₋₄ alkoxy, nitro or cyano for preparing a pharmaceutical preparation for the therapy of psoriasis, psoriatic arthritis, neurodermitis and enteritis regionalis Crohn, as well as of autoimmune diseases selected from the group consisting of polyarthritis, multipole sclerosis, juvenile-onset diabetes, Hashimoto's thyroiditis, Grave's disease, systemic Lupus erythematodes (SLE), Sjogren's syndrome, pernicious anaemia and chronic active (lupoid) hepatitis.

17. The use according to claim 16, **characterised in that** the radicals R₁ and R₂ are methyl, ethyl, n-propyl, isopropyl, n-butyl, sec-butyl, t-butyl, pentyl, cyclopentyl, 2-ethyl hexyl, hexyl, cyclohexyl, heptyl, cycloheptyl, octyl, vinyl, allyl, 2-hydroxy ethyl, 2 and/or 3-hydroxy propyl, 2-methoxy ethyl, methoxy ethyl or 2- or 3-methoxy propyl.

18. The use according to claim 15 or 16, **characterised in that** R₁ and R₂ are identical and represent methyl or ethyl.

19. The use according to any of the claims 15 to 18 where the active ingredients are formulated into an oral preparation in the form of tablets, micro-tablets, pellets in capsules or sachets.

20. The use according to any of the claims 15 to 19, **characterised in that** the size or the mean diameter, respectively, of the pellets or micro-tablets is in the range of 300 to 2,000 µm.

21. The use according to claim 19, **characterised in that** the preparation is present in the form of soft or hard gelatine capsules.

22. The use according to any of the claims 15 to 21 where the preparation contains an amount of the active ingredient corresponding to 10 to 300 mg of fumaric acid.

23. The use according to claims 19 to 22, **characterised in that** the microtablets or pellets are provided with an enteric coating.

## Revendications

1. Utilisation d'un ou de plusieurs fumarates de dialkyle pour la préparation d'un médicament pour le traitement des réactions de l'hôte contre le greffon ou du greffon contre l'hôte dans les transplantations d'organes et de cellules.

2. Utilisation d'un ou de plusieurs fumarates de dialkyle pour la préparation d'un médicament pour le traitement de maladies auto-immunes choisies dans le groupe constitué par le diabète juvénile, la thyroïdite de Hashimoto, « Grave's disease » (maladie de Grave ou maladie de Basedow), le lupus érythémateux systémique (SLE), le syndrome de Sjögren (Sjögren's Syndrome), l'anémie pernicieuse et l'hépatite chronique active (= lupoïde).

3. Utilisation selon la revendication 1 ou 2 d'un ou plusieurs fumarates de dialkyle de formule : 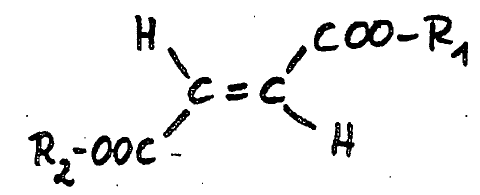 dans laquelle R₁ et R₂, qui peuvent être identiques ou différents, représentent, indépendamment l'un de l'autre, un groupe alkyle en C₁ à C₂₀ linéaire, ramifié ou cyclique, saturé ou insaturé, qui peut éventuellement être substitué par un halogène (Cl, F, I, Br), hydroxy, alcoxy en C₁ à C₄, nitro ou cyano, pour la préparation d'un médicament pour l'utilisation dans la médecine des transplantations ou pour la thérapie de maladies auto-immunes choisies dans le groupe constitué par le diabète juvénile, la thyroïdite de Hashimoto, « Grave's disease » (maladie de Grave ou maladie de Basedow), le lupus érythémateux systémique (SLE), le syndrome de Sjögren (Sjögren's Syndrome), l'anémie pernicieuse et l'hépatite chronique active (= lupoïde).

4. Utilisation selon la revendication 3, **caractérisée en ce qu'**il s'agit pour les groupes R₁ et R₂ du groupe méthyle, éthyle, n-propyle, isopropyle, n-butyle, sec.-butyle, t-butyle, pentyle, cyclopentyle, 2-éthylhexyle, hexyle, cyclohexyle, heptyle, cycloheptyle, octyle, vinyle, allyle, 2-hydroxyéthyle, 2- et/ou 3-hydroxypropyle, 2-méthoxyéthyle, méthoxyéthyle ou 2- ou 3-méthoxypropyle.

5. Utilisation selon la revendication 3 ou 4, **caractérisée en ce que** R₁ et R₂ sont identiques et représentent un méthyle ou éthyle.

6. Utilisation selon une des revendications 1 à 5, dans laquelle les substances actives pour une préparation orale sont formulées sous forme de comprimés, de microcomprimés, de pastilles ou de granulés éventuellement en capsules ou sachets.

7. Utilisation selon la revendication 6, **caractérisée en ce que** la taille ou le diamètre moyen de pastilles ou de microcomprimés se situe dans la gamme de 300 à 2 000 µm.

8. Utilisation selon les revendications 1 à 7, **caractérisée en ce que** la préparation se présente sous la forme de capsules de gélatine molle ou dure.

9. Utilisation selon une des revendications 1 à 8, dans laquelle la préparation contient une quantité de substance active correspondant à 10 à 300 mg d'acide fumarique.

10. Utilisation selon les revendications 6 à 9, **caractérisée en ce que** les unités de dose des médicaments sont munies d'un revêtement résistant au suc gastrique.

11. Utilisation selon les revendications 1 à 5, **caractérisée en ce qu'**on utilise les médicaments sous la forme de préparations pour l'administration cutanée et transdermique, de préparations pour l'administration parentérale et de préparations pour l'administration rectale.

12. Composition pharmaceutique sous la forme de microcomprimés ou de pastilles, constitué d'un ou de plusieurs fumarates de dialkyle de formule : 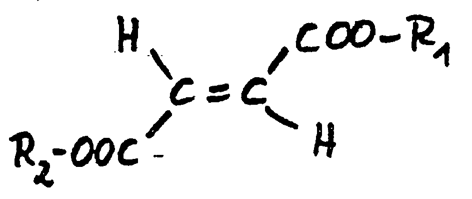 dans laquelle R₁ et R₂, qui peuvent être identiques ou différents, représentent, indépendamment l'un de l'autre, un groupe alkyle en C₁ à C₂₀ linéaire, ramifié ou cyclique, saturé ou insaturé, qui peut éventuellement être substitué par un halogène (Cl, F, I, Br), hydroxy, alcoxy en C₁ à C₄, nitro ou cyano, et éventuellement de supports et adjuvants appropriés.

13. Préparation selon la revendication 12, contenant du fumarate de diméthyle, du fumarate de diéthyle ou du fumarate de méthyle et d'éthyle.

14. Préparation selon la revendication 12 ou 13, contenant une quantité de substance active correspondant à 10 à 300 mg d'acide fumarique.

15. Utilisation d'un ou de plusieurs fumarates de dialkyle pour la préparation d'un médicament sous la forme de microcomprimés ou de pastilles pour la thérapie du psoriasis, de l'arthrite-psoriasique, de la neurodermite, de l'entérite, régionale de Crohn ainsi que de maladies auto-immunes choisies dans le groupe constitué par la polyarthrite, les scléroses multiples, le diabète juvénile, la thyroïdite de Hashimoto, a « Grave's disease » (maladie de Grave ou maladie de Basedow), le lupus érythémateux systémique (SLE), le syndrome de Sjögren (Sjögren's Syndrome), l'anémie pernicieuse et l'hépatite chronique active (= lupoïde).

16. Utilisation selon la revendication 15 d'un ou de plusieurs fumarates de dialkyle de formule : 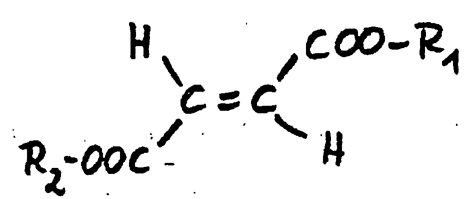 dans laquelle R₁ et R₂, qui peuvent être identiques ou différents, représentent, indépendamment l'un de l'autre, un groupe alkyle en C₁ à C₂₀ linéaire, ramifié ou cyclique, saturé ou insaturé, qui peut éventuellement être substitué par un halogène (Cl, F, I, Br), hydroxy, alcoxy en C₁ à C₄, nitro ou cyano, pour la préparation d'un médicament pour la thérapie du psoriasis, de l'arthrite psoriasique, de la neurodermite, de l'entérite régionale de Crohn ainsi que de maladies auto-immunes choisies dans le groupe constitué par la polyarthrite, les scléroses multiples, le diabète juvénile, la thyroïdite de Hashimoto, « Grave's disease » (maladie de Grave ou maladie de Basedow), le lupus érythémateux systémique (SLE), le syndrome de Sjögren (Sjögren's Syndrome), l'anémie pernicieuse et l'hépatite chronique active (= lupoïde).

17. Utilisation selon la revendication 16, **caractérisée en ce qu'**il s'agit pour les groupes R₁ et R₂ du groupe méthyle, éthyle, n-propyle, isopropyle, n-butyle, sec.-butyle, t-butyle, pentyle, cyclopentyle, 2-éthylhexyle, hexyle, cyclohexyle, heptyle, cycloheptyle, octyle, vinyle, allyle, 2-hydroxyéthyle, 2- et/ou 3-hydroxypropyle, 2-méthoxyéthyle, méthoxyéthyle ou 2- ou 3-méthoxypropyle.

18. Utilisation selon la revendication 15 ou 16, **caractérisée en ce que** R₁ et R₂ sont identiques et représentent un méthyle ou éthyle.

19. Utilisation selon une des revendications 15 à 18, dans laquelle les substances actives pour une administration orale sont formulées sous forme de microcomprimés, ou de pastilles en capsules ou sachets.

20. Utilisation selon l'une des revendications 15 à 19, **caractérisée en ce que** la taille ou le diamètre moyen de pastilles ou de microcomprimés se situe dans la gamme de 300 à 2 000 µm.

21. Utilisation selon la revendication 19, **caractérisée en ce que** la préparation se présente sous la forme de capsules de gélatine molle ou dure.

22. Utilisation selon une des revendications 15 à 21, dans laquelle la préparation contient une quantité de substance active correspondant à 10 à 300 mg d'acide fumarique.

23. Utilisation selon les revendications 19 à 22, **caractérisée en ce que** les pastilles ou les microcomprimés sont munis d'un revêtement résistant au suc gastrique.
